# EUROPÄISCHE PATENTSCHRIFT

(11) **EP 2 724 686 B1**
(45) Veröffentlichungstag und Bekanntmachung des Hinweises auf die Patenterteilung: **28.06.2017**
(21) Anmeldenummer: 13181834.6
(22) Anmeldetag: 27.08.2013
(51) Int. Cl.: A61C 8/00

(54) **Dentalimplantat und dentales Implantatsystem**
Dental implant and dental implant system
Implant dentaire et système d'implant dentaire

(30) Priorität: 29.10.2012 DE 102012110318
(43) Veröffentlichungstag der Anmeldung: 30.04.2014
(73) Patentinhaber: DENTAURUM GmbH & Co. KG, 75228 Ispringen (DE)
(72) Erfinder: Grosse, Tobias, 75228 Ispringen (DE); Bauer, Andreas, 76646 Bruchsal (DE); Heinemann, Friedhelm, 51580 Reichshof (DE)
(74) Vertreter: Hoeger, Stellrecht & Partner Patentanwälte mbB

(56) Entgegenhaltungen:
- EP-A1- 2 347 729
- WO-A2-01/49199
- DE-U1-202005 000 208

## Beschreibung

Die vorliegende Erfindung betrifft ein dentales Implantatsystem umfassend ein Dentalimplantat mit einer Aufnahme für dentale Aufbauteile, wobei sich die Aufnahme entlang einer Längsachse des Dentalimplantats erstreckt und ein dentales Aufbauteil axial in die Aufnahme einführbar ist, und mindestens ein erstes und mindestens ein zweites dentales Aufbauteil, die wahlweise mit dem Dentalimplantat verbindbar sind, wobei die Aufbauteile jeweils einen Verbindungsbereich umfassen, der axial in die Aufnahme des Dentalimplantats einführbar ist. Ein derartiges Implantatsystem ist aus der EP 2 347 729 A1 bekannt. In der dentalen Implantologie werden fast immer mehrteilige Anordnungen verwendet, die zum einen das eigentliche Dentalimplantat umfassen, welches zum dauerhaften Verbleib in den Kieferknochen implantiert wird. Das Implantat weist eine Verbindungsstelle zur Anordnung eines dentalen Aufbauteils auf, wobei das Implantat im Verlauf der implantologischen Behandlung typischerweise mit einer Reihe von verschiedenen Aufbauteilen kombiniert wird: So kann z.B. während der Heilungsphase nach dem Einsetzen des Implantats die Verbindungsstelle zunächst durch ein Verschlusselement gegen das Eindringen von Verunreinigungen geschützt werden. Anschließend wird das Implantat in der Regel mit einem Abdruckpfosten verbunden, der dazu dient, bei der Anfertigung eines Gebissabdrucks die exakte Position und Ausrichtung des Dentalimplantats zu ermitteln. Diese Information benötigt der Zahntechniker, um einen Zahnersatz (z.B. eine Endprothese), der letztlich mit dem Implantat verbunden werden soll, mit der nötigen Präzision herstellen zu können. Der Zahnersatz wird mittels eines als Abutment bezeichneten dentalen Aufbauteils mit dem Dentalimplantat verbunden. Weitere Aufbauteile, die mit einem Dentalimplantat verbunden werden können, sind z.B. Implantathalterungen und Gingivaformer.

Die Verbindung zwischen dem Dentalimplantat und dem Aufbauteil kann prinzipiell als Innenverbindung oder als Außenverbindung ausgestaltet sein, wobei die vorliegende Erfindung Dentalimplantate mit einer Innenverbindung betrifft, bei denen das Aufbauteil in eine Aufnahme, die innerhalb des Implantats ausgebildet ist, eingeführt wird. Bei dieser Art der Verbindung sind aus dem Stand der Technik prinzipiell zwei Varianten bekannt, nämlich eine zylindrische Innenverbindung und eine konische Innenverbindung, wobei beide Varianten jeweils Vor- und Nachteile aufweisen.

Bei der zylindrischen Innenverbindung weist die Aufnahme des Dentalimplantats einen zylindrischen Wandabschnitt auf, der zur formschlüssigen Aufnahme eines korrespondierenden zylindrischen Verbindungsbereichs des Aufbauteils dient. Zur Festlegung der axialen Position des Aufbauteils in Bezug auf das Implantat dient in diesem Fall eine zur Längsachse senkrechte Auflagefläche, an der sich das Aufbauteil abstützt. Die durch die Auflagefläche festgelegte Position des Aufbauteils ist sehr exakt, da sie durch Fertigungstoleranzen praktisch nicht beeinflusst wird, was den wesentlichen Vorteil der zylindrischen Innenverbindung darstellt. Insbesondere kann dadurch sichergestellt werden, dass die axiale Position eines Abdruckpfostens exakt der späteren axialen Position eines Abutments entspricht, sodass eine diesbezügliche Nachbearbeitung des vom Zahntechniker hergestellten Zahnersatzes nicht erforderlich ist. Die exakt definierte Auflagefläche erleichtert auch die Umsetzbarkeit mittels CAD/CAM-Technik. Nachteilig bei der zylindrischen Innenverbindung ist, dass sich bei einer sehr hohen transversalen Krafteinleitung in das Aufbauteil ein Spalt zwischen dem Implantat und dem Aufbauteil bilden kann, da die Passung des Aufbauteils in dem zylindrischen Wandabschnitt der Aufnahme nicht vollständig spielfrei ausgebildet werden kann.

Bei der konischen Innenverbindung weist die Aufnahme des Dentalimplantats einen kegelstumpfförmigen Wandabschnitt auf, der sich von koronal nach apikal verjüngt. In diesen Konus wird ein korrespondierender kegelstumpfförmiger Verbindungsbereich des dentalen Aufbauteils eingeführt, sodass sowohl die radiale als auch die axiale Position des Aufbauteils durch das formschlüssige Ineinandergreifen der korrespondierenden Konen definiert wird. Vorteile dieser Art der Innenverbindung sind die gute Kraftübertragung vom Aufbauteil auf das Implantat, die mit einer wesentlich geringeren Spaltbildung bei hohen transversalen Kräften verbunden ist, sowie die erleichterte Handhabung beim Einführen des schmäleren apikalen Endes des Aufbauteils in die Aufnahme des Implantats. Nachteilig ist andererseits, dass die axiale Position des Aufbauteils in wesentlich höherem Maße durch Fertigungstoleranzen beeinflusst wird und daher weniger exakt ist als bei der zylindrischen Innenverbindung. Da die konische Verbindung nicht nur formschlüssig, sondern ggf. auch reibschlüssig ist, ist die Position des Aufbauteils auch von der axialen Kraftbeaufschlagung abhängig.

In Abwägung der jeweiligen Vor- und Nachteile muss sich der Zahnarzt vor Beginn einer implantologischen Behandlung für ein System mit einer zylindrischen oder einer konischen Innenverbindung entscheiden, und ein entsprechendes Implantat einsetzen. Ein Wechsel des Verbindungssystems ist danach bei den Implantaten gemäß dem Stand der Technik nicht mehr möglich. Dies wäre jedoch häufig wünschenswert, da z.B. bei verschiedenen Arbeitsschritten einmal eine zylindrische und einmal eine konische Verbindung vorteilhafter sein kann.

Der vorliegenden Erfindung liegt die Aufgabe zugrunde, ein dentales Implantatsystem vorzuschlagen, welches dem Anwender eine höhere Flexibilität bei der Wahl des Verbindungstyps zwischen dem Implantat und einem dentalen Aufbauteil ermöglicht.

Diese Aufgabe wird erfindungsgemäß durch ein dentales Implantatsystem nach Anspruch 1 gelöst. Das Dentalimplantat des erfindungsgemäßen dentalen Implantatsystems bietet somit die vorteilhafte Möglichkeit, wahlweise über eine zylindrische oder eine konische Innenverbindung mit einem entsprechenden dentalen Aufbauteil verbunden zu werden. Der Zahnarzt kann sich also nach dem Einsetzen des Implantats in den Kieferknochen für einen der Verbindungstypen entscheiden, d.h. er kann bei dieser Entscheidung auch Umstände berücksichtigen, die sich erst während oder nach der Implantation ergeben haben. Er kann insbesondere die prothetische Versorgung jederzeit indikationsbedingt erweitern oder modifizieren, und in Abhängigkeit von dieser Erweiterung einen optimalen Verbindungstyp (konisch oder zylindrisch) wählen. So kann z.B. ein vor Jahren gesetztes Einzelzahnimplantat mit einer konischen Verbindung des Aufbauteils problemlos in eine gefräste Stegversorung mit einer zylindrischen Verbindung des Aufbauteils integriert werden.

Günstigerweise sind die zylindrischen und kegelstumpfförmigen Wandabschnitte jeweils rotationssymmetrisch in Bezug auf die Längsachse des Dentalimplantats ausgebildet und in Axialrichtung aufeinanderfolgend angeordnet. Beispielsweise kann in Axialrichtung von koronal nach apikal ein zylindrischer auf einen kegelstumpfförmigen Wandabschnitt folgen oder umgekehrt. Die verschiedenen Wandabschnitte sind jedoch nicht unmittelbar aufeinanderfolgend, d.h. zwischen einem zylindrischen und einem kegelstumpfförmigen Wandabschnitt ist ein als Rotationssicherung dienender Abschnitt der Aufnahme angeordnet, dessen Wandung weder zylindrisch noch kegelstumpfförmig ist.

Bei der Verbindung des Dentalimplantats mit dem ersten Aufbauteil wird der mindestens eine zylindrische Wandabschnitt und die senkrechte Auflagefläche genutzt, nicht jedoch der mindestens eine kegelstumpfförmige Wandabschnitt (zylindrische Innenverbindung). Bei der Verbindung des Dentalimplantats mit dem zweiten Aufbauteil wird der mindestens eine kegelstumpfförmige Wandabschnitt genutzt, nicht jedoch die senkrechte Auflagefläche (konische InnenVerbindung). Es ist jedoch möglich, dass bei der konischen Innenverbindung zusätzlich auch der mindestens eine zylindrische Wandabschnitt zur radialen Abstützung des Aufbauteils genutzt wird.

Bei einer bevorzugten Ausführungsform der Erfindung umfasst die Aufnahme einen zylindrischen Wandabschnitt im Bereich ihres apikalen Endes und einen kegelstumpfförmigen Wandabschnitt im Bereich ihres koronalen Endes. Der Durchmesser der Aufnahme im Bereich des zylindrischen Wandabschnitts ist in diesem Fall kleiner als der Durchmesser der Aufnahme im Bereich des koronalen Endes des kegelstumpfförmigen Wandabschnitts, und bevorzugt auch kleiner als der Durchmesser im Bereich des apikalen Endes des kegelstumpfförmigen Wandabschnitts. Zusätzlich zu diesen beiden Wandabschnitten kann die Aufnahme noch weitere zylindrische und/oder kegelstumpfförmige Wandabschnitte umfassen.

Die senkrechte Auflagefläche erstreckt sich vorzugsweise vom koronalen Ende der Aufnahme nach außen. Die Auflagefläche kann sich z.B. unmittelbar an einen kegelstumpfförmigen Wandabschnitt am koronalen Ende der Aufnahme anschließen. Die Auflagefläche ist in diesem Fall günstigerweise ringförmig ausgebildet, sie kann nach außen aber z.B. auch eine polygonale Begrenzung aufweisen, je nach Gestaltung der Außenkontur des Dentalimplantats.

Alternativ kann die senkrechte Auflagefläche auch durch eine apikale Bodenfläche der Aufnahme gebildet werden. In diesem Fall ist es besonders vorteilhaft, wenn sich diese Bodenfläche an einen zylindrischen Wandabschnitt am apikalen Ende der Aufnahme anschließt.

Bei einer weiteren vorteilhaften Ausführungsform der Erfindung umfasst die Aufnahme zwei zylindrische Wandabschnitte, zwischen denen ein kegelstumpfförmiger Wandabschnitt angeordnet ist. Die beiden zylindrischen Wandabschnitte weisen in diesem Fall unterschiedliche Durchmesser auf, wobei der Durchmesser des apikalen Wandabschnitts naturgemäß kleiner ist. Durch das Vorsehen mehrerer zylindrischer Wandabschnitte kann die Stabilität der Verbindung zwischen dem Dentalimplantat und dem ersten dentalen Aufbauteil erhöht werden. Zusätzlich kann vorgesehen sein, dass die Aufnahme zwei kegelstumpfförmige Wandabschnitte umfasst, zwischen denen ein zylindrischer Wandabschnitt angeordnet ist. In diesem Fall ist es besonders vorteilhaft, wenn die zwei kegelstumpfförmigen Wandabschnitte auf derselben gedachten Kegelfläche liegen, da eine solche Konfiguration relativ einfach mit hoher Präzision herstellbar ist.

Der mindestens eine kegelstumpfförmige Wandabschnitt weist bevorzugt einen Neigungswinkel zur Längsachse im Bereich von ca. 5° bis ca. 15° auf. Je kleiner der Neigungswinkel ist, desto eher kann auch eine kraftschlüssige Verbindung mit dem zweiten dentalen Aufbauteil nach Art eines selbsthemmenden Konus erreicht werden, desto mehr ist jedoch auch die axiale Position des zweiten Aufbauteils von der axialen Krafteinwirkung abhängig. Erfindungsgemäß umfasst die Aufnahme einen als Rotationssicherung dienenden Abschnitt, in den ein korrespondierender Abschnitt eines dentalen Aufbauteils eingreifen kann. Durch eine solche Rotationssicherung kann eine definierte Winkelposition des dentalen Aufbauteils in Bezug auf das Dentalimplantat festgelegt werden. Der als Rotationssicherung dienende Abschnitt kann auf verschiedene Weise ausgebildet sein, insbesondere durch entlang des Umfangs verteilte Rücksprünge in der Wandung der Aufnahme, in die korrespondierende Vorsprünge an dem Verbindungsbereich des dentalen Aufbauteils eingreifen.

Der als Rotationssicherung dienende Abschnitt ist zwischen einem zylindrischen und einem kegelstumpfförmigen Wandabschnitt der Aufnahme angeordnet. Beispielsweise kann die Aufnahme von koronal nach apikal einen kegelstumpfförmigen Wandabschnitt, einen als Rotationssicherung dienenden Abschnitt und einen zylindrischen Wandabschnitt umfassen, wobei sich die senkrechte Auflagefläche vom koronalen Ende des kegelstumpfförmigen Wandabschnitts nach außen erstreckt.

Das mit dem Dentalimplantat verbundene Aufbauteil wird üblicherweise durch ein Befestigungselement, z.B. eine Schraube, an dem Dentalimplantat fixiert, insbesondere, wenn es sich bei dem Aufbauteil um ein Abutment handelt, welches dauerhaft und möglichst fest mit dem Implantat verbunden werden soll. Zu diesem Zweck ist es bevorzugt, wenn das Dentalimplantat eine sich apikal an die Aufnahme anschließende Gewindebohrung umfasst, in die eine Schraube zur Fixierung des Aufbauteils eingeschraubt werden kann. Entsprechende erste und zweite Aufbauteile weisen dann eine axiale Bohrung auf, durch die die Schraube hindurchtritt.

Das Einsetzen des Dentalimplantats in den Kieferknochen kann auf verschiedene Arten erfolgen, die an sich aus dem Stand der Technik bekannt sind. Gemäß einer Ausführungsform der Erfindung ist das Dentalimplantat in Form einer Knochenschraube mit einem Außengewinde zum Einschrauben in einen Kieferknochen ausgebildet.

Das Dentalimplantat kann aus verschiedenen Materialien gebildet sein, die für den Einsatz in der dentalen Implantologie geeignet sind. Vorzugsweise ist das Dentalimplantat ganz oder teilweise aus Titan, einer Titanlegierung, einer Cobalt-Chrom-Legierung oder Keramik gebildet.

Die Vorteile des erfindungsgemäßen Implantatsystems wurden im Wesentlichen bereits im Zusammenhang mit dem Dentalimplantat beschrieben, wobei der Grundgedanke der Erfindung darin liegt, dass innerhalb dieses Systems ein- und dasselbe Dentalimplantat wahlweise mit einem ersten Aufbauteil über eine zylindrische Innenverbindung oder mit einem zweiten Aufbauteil über eine konische Innenverbindung verbunden werden kann. Günstigerweise umfasst das Implantatsystem mehrere erste Aufbauteile und mehrere zweite Aufbauteile, deren Verbindungsbereiche jeweils gleich ausgebildet sind, die sich jedoch in ihrer grundlegenden Funktion unterscheiden. Das System kann also beispielsweise ein Abutment, einen Abdruckpfosten usw. mit einem Verbindungsbereich für eine zylindrische Verbindung sowie ein Abutment, einen Abdruckpfosten usw. mit einem Verbindungsbereich für eine konische Verbindung umfassen. Erfindungsgemäß umfasst der Verbindungsabschnitt des mindestens einen zweiten Aufbauteils zusätzlich einen zylindrischen Wandabschnitt, der mit dem korrespondierenden zylindrischen Wandabschnitt der Aufnahme formschlüssig ineinander greift, wenn das Aufbauteil mit dem Implantat verbunden wird. Der zylindrische Wandabschnitt der Aufnahme kann also sowohl für das erste als auch für das zweite Aufbauteil zur radialen Abstützung des korrespondierenden Wandabschnitts des Verbindungsbereichs genutzt werden, wie bereits oben beschrieben wurde. Entscheidend für den Unterschied zwischen den beiden Arten von Aufbauteilen ist jedoch, dass bei dem mindestens einen zweiten Aufbauteil keine korrespondierende Gegenfläche vorhanden ist, die sich an der zur Längsachse senkrechten Auflagefläche des Dentalimplantats abstützen könnte.

Bei einer weiteren Ausführungsform des erfindungsgemäßen Implantatsystems umfasst der Verbindungsbereich des mindestens einen ersten Aufbauteils zwei zylindrische Wandabschnitte und/oder der Verbindungsbereich des mindestens einen zweiten Aufbauteils umfasst zwei kegelstumpfförmige Wandabschnitte. In diesem Fall umfasst die Aufnahme des Dentalimplantats ebenfalls zwei jeweils korrespondierende Wandabschnitte, wie dies bereits oben erläutert wurde.

Bevorzugt umfasst der Verbindungsbereich des mindestens einen ersten Aufbauteils und/oder des mindestens einen zweiten Aufbauteils jeweils einen als Rotationssicherung dienenden Abschnitt, der mit einem korrespondierenden Abschnitt der Aufnahme des Dentalimplantats formschlüssig ineinander greift, wenn das Aufbauteil mit dem Dentalimplantat verbunden wird. Die Rotationssicherung verhindert eine Verdrehung des Aufbauteils um die Längsachse und ermöglicht eine exakte Festlegung der Winkelposition in Bezug auf das Dentalimplantat. Mögliche Ausgestaltungen der Rotationssicherung wurden ebenfalls bereits im Zusammenhang mit dem erfindungsgemäßen Dentalimplantat beschrieben.

Diese und weitere Vorteile der Erfindung werden anhand des nachfolgenden Ausführungsbeispiels unter Bezugnahme auf die Figuren näher erläutert.

Es zeigen im Einzelnen:
- Figur 1:: Perspektivische Darstellung eines Dentalimplantats eines erfindungsgemäßen dentalen Implantatsystems;
- Figur 2:: Perspektivische Darstellung eines ersten dentalen Aufbauteils eines erfindungsgemäßen dentalen Implantatsystems;
- Figur 3:: Perspektivische Darstellung eines zweiten dentalen Aufbauteils eines erfindungsgemäßen dentalen Implantatsystems;
- Figur 4:: Darstellung des Dentalimplantats gemäß Figur 1 und des ersten Aufbauteils gemäß Figur 2 im Längsschnitt; und
- Figur 5:: Darstellung des Dentalimplantats gemäß Figur 1 und des zweiten Aufbauteils gemäß Figur 3 im Längsschnitt.

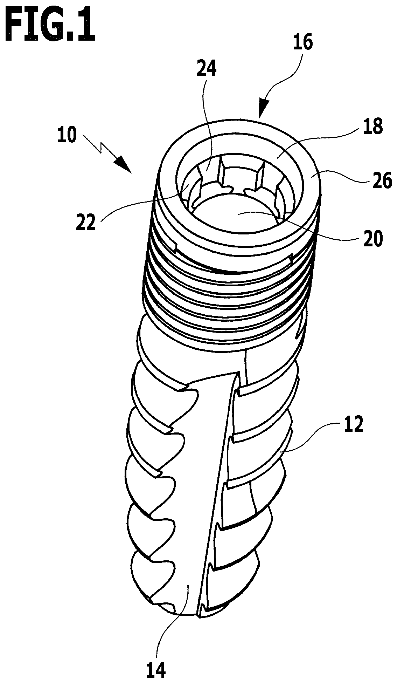

Die Figur 1 zeigt eine perspektivische Darstellung eines Ausführungsbeispiels eines Dentalimplantats eines erfindungsgemäßen dentalen Implantatsystems, welches insgesamt mit 10 bezeichnet ist. Das Dentalimplantat 10 ist als Knochenschraube ausgebildet und weist ein Außengewinde 12 mit einer Quernut 14 auf. Das Dentalimplantat 10 erstreckt sich entlang einer Längsachse, wobei in der Figur 1 das koronale Ende oben und das apikale Ende unten angeordnet ist.

Das Dentalimplantat 10 weist eine Aufnahme 16 auf, die sich vom koronalen Ende entlang der Längsachse in das Dentalimplantat 10 erstreckt. Die Aufnahme 16 umfasst verschiedene Wandabschnitte, die jeweils rotationssymmetrisch in Bezug auf die Längsachse ausgebildet sind, und zwar im Bereich des koronalen Endes einen kegelstumpfförmigen Wandabschnitt 18 und im Bereich des apikalen Endes einen zylindrischen Wandabschnitt 20. Zwischen diesen beiden Wandabschnitten ist ein als Rotationssicherung dienender Abschnitt 22 angeordnet, der entlang des Umfangs der Aufnahme 16 angeordnete Rücksprünge 24 aufweist.

Das Dentalimplantat 10 weist außerdem eine zur Längsachse senkrechte Auflagefläche 26 auf, die sich vom koronalen Ende der Aufnahme 16 nach außen erstreckt. Die Auflagefläche 26 ist ringförmig ausgebildet.

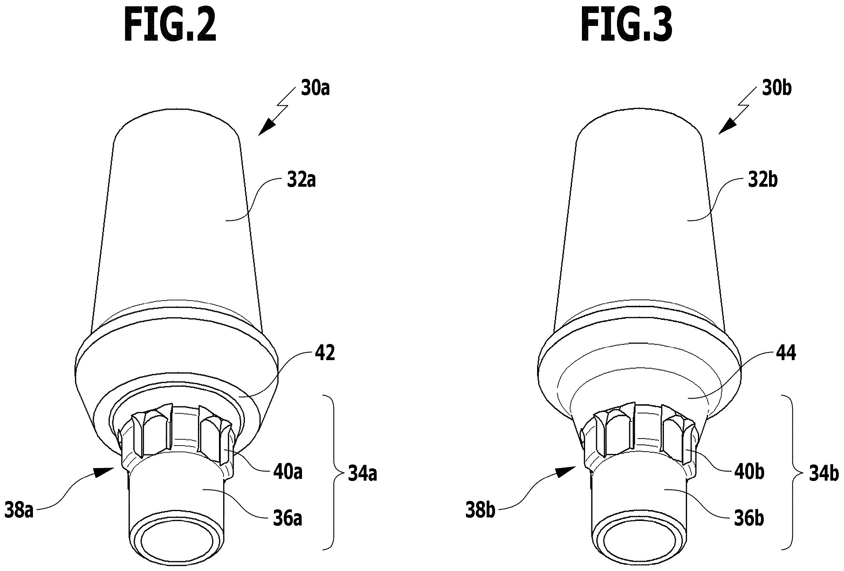

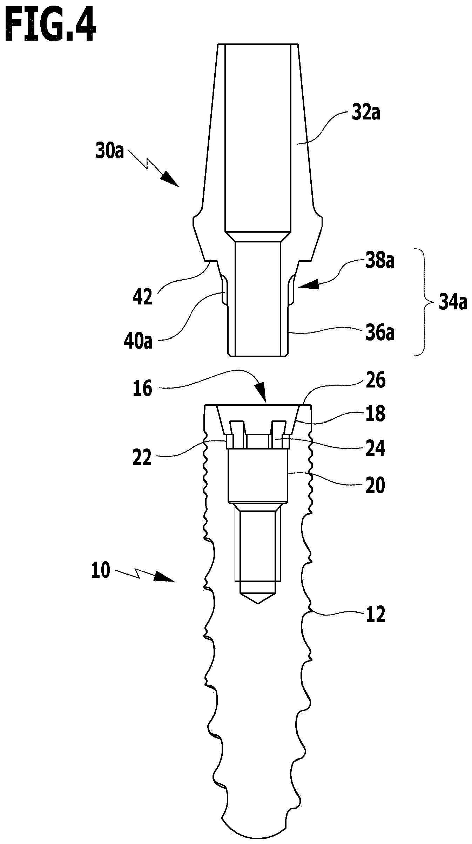

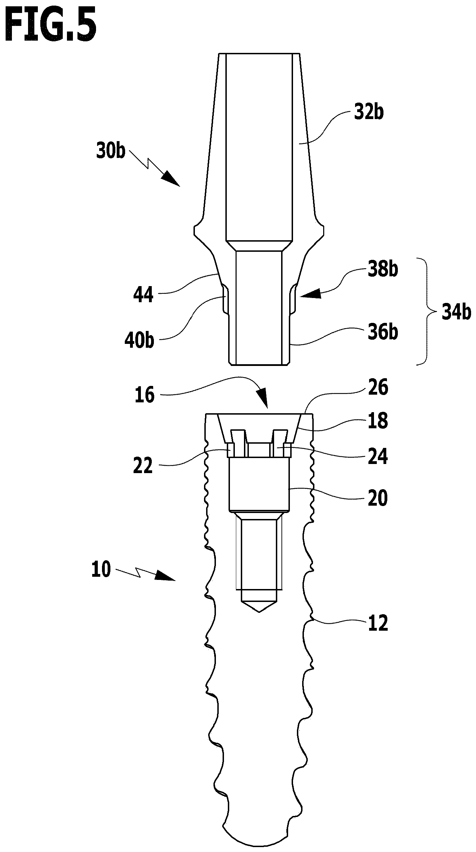

Ein dentales Implantatsystem gemäß der vorliegenden Erfindung umfasst neben dem Dentalimplantat gemäß der Figur 1 mindestens ein erstes dentales Aufbauteil, das über eine zylindrische Verbindung mit dem Dentalimplantat 10 verbindbar ist, und mindestens ein zweites dentales Aufbauteil, das über eine konische Verbindung mit dem Dentalimplantat 10 verbindbar ist. Ein Ausführungsbeispiel eines ersten Aufbauteils 30a ist in der Figur 2 perspektivisch dargestellt, und die Figur 4 zeigt einen Längsschnitt durch das Dentalimplantat 10 und das erste Aufbauteil 30a. Ein Ausführungsbeispiel eines zweiten Aufbauteils 30b ist in der Figur 3 perspektivisch dargestellt, und die Figur 5 zeigt einen Längsschnitt durch das Dentalimplantat 10 und das zweite Aufbauteil 30b.

Bei dem ersten Aufbauteil 30a handelt es sich um ein Abutment mit einem koronalen Trägerbereich 32a, auf den z.B. eine Endprothese aufgebracht werden kann, und mit einem apikalen Verbindungsbereich 34a zur Verbindung des Aufbauteils 30a mit dem Dentalimplantat 10. Der Verbindungsbereich 34a wird hierbei axial in die Aufnahme 16 eingeführt.

Der Verbindungsbereich 34a des ersten Aufbauteils 30a umfasst an seinem apikalen Ende einen zylindrischen Wandabschnitt 36a und einen sich daran anschließenden Abschnitt 38a, der als Rotationssicherung dient. Die Rotationssicherung 38a umfasst entlang des Umfangs des Verbindungsbereichs 34a angeordnete Vorsprünge 40a. Ferner umfasst der Verbindungsbereich 34a eine zur Längsachse senkrechte Auflagefläche 42, die ringförmig ausgebildet und in Apikalrichtung orientiert ist.

Beim Verbinden des ersten Aufbauteils 30a mit dem Dentalimplantat 10 wird der Verbindungsbereich 34a axial in die Aufnahme 16 eingeführt, wobei die korrespondierenden zylindrischen Wandabschnitte 20 und 36a ineinander greifen und die Vorsprünge 40a der Rotationssicherung 38a in die Rücksprünge 24 der Rotationssicherung 22 eingreifen. Die Einführungstiefe wird dadurch begrenzt, dass die Auflageflächen 26 und 42 aneinander anliegen. Somit dienen die zylindrischen Wandabschnitte 20 und 36a der radialen Abstützung und die Auflageflächen 26 und 42 der axialen Abstützung des ersten Aufbauteils 30a. Durch die als Rotationssicherung dienenden Abschnitte 22 und 38a wird die Winkelposition des Aufbauteils 30a in Bezug auf das Dentalimplantat 10 festgelegt.

Bei dem zweiten dentalen Aufbauteil 30b handelt es sich ebenfalls um ein Abutment mit einem Trägerbereich 32b und einem Verbindungsbereich 34b. Der Verbindungsbereich 34b umfasst einen apikalen zylindrischen Wandabschnitt 36b und einen sich daran anschließenden, als Rotationssicherung dienenden Abschnitt 38b. Die Abschnitte 36b und 38b des zweiten Aufbauteils 30b sind wie die entsprechenden Abschnitte 36a und 38a des ersten Aufbauteils 30a ausgebildet. An seinem koronalen Ende umfasst der Verbindungsbereich 34b des zweiten Aufbauteils 30b einen kegelstumpfförmigen Wandabschnitt 44, der sich in Apikalrichtung verjüngt.

Beim Verbinden des zweiten Aufbauteils 30b mit dem Dentalimplantat 10 wird der Verbindungsbereich 34b axial in die Aufnahme 16 eingeführt, wobei die korrespondierenden zylindrischen Wandabschnitte 20 und 36b sowie die korrespondierenden Rotationssicherungen 22 und 38b auf dieselbe Weise ineinander greifen wie bei dem ersten Aufbauteil 30a. Die Einführungstiefe des Verbindungsabschnitts 34b wird in diesem Fall begrenzt durch das formschlüssige und gegebenenfalls reibschlüssige Ineinandergreifen der korrespondierenden kegelstumpfförmigen Wandabschnitte 18 und 44, wobei die senkrechte Auflagefläche 16 des Dentalimplantats 10 nicht mit einer korrespondierenden Gegenfläche in Kontakt kommt. Die kegelstumpfförmigen Wandabschnitte 18 und 44 dienen somit der axialen und radialen Abstützung des zweiten Aufbauteils 30b, und die korrespondierenden zylindrischen Wandabschnitte 20 und 36b dienen der zusätzlichen radialen Abstützung.

Das erfindungsgemäße dentale Implantatsystem gemäß den Figuren 1 bis 5 kann zusätzlich weitere erste und zweite Aufbauteile umfassen, deren Verbindungsbereich identisch ausgebildet ist wie bei den Aufbauteilen 30a oder 30b, die jedoch anstelle des Trägerbereichs 32a oder 32b eine andere Gestaltung aufweisen, also nicht als Abutments, sondern z.B. als Abdruckpfosten oder Gingivaformer ausgebildet sind. Bei einem derartigen System kann der Zahnarzt für jeden Behandlungsschritt einer implantologischen Behandlung individuell entscheiden, ob er ein erstes Aufbauteil mit einem Verbindungsbereich 34a für eine zylindrische Verbindung oder ein zweites Aufbauteil mit einem Verbindungsbereich 34b für eine konische Verbindung einsetzt, je nachdem, ob eine möglichst exakte axiale Positionierung des Aufbauteils oder eine möglichst gute Kraftübertragung vom Aufbauteil auf das Dentalimplantat im Vordergrund steht.

### Bezugszeichenliste

- 10: Dentalimplantat
- 12: Außengewinde
- 14: Quernut
- 16: Aufnahme
- 18: Kegelstumpfförmiger Wandabschnitt
- 20: Zylindrischer Wandabschnitt
- 22: Rotationssicherung
- 24: Rücksprung
- 26: Senkrechte Auflagefläche
- 30a: Erstes dentales Aufbauteil
- 32a: Trägerbereich
- 34a: Verbindungsbereich
- 36a: Zylindrischer Wandabschnitt
- 38a: Rotationssicherung
- 40a: Vorsprung
- 42: Senkrechte Auflagefläche
- 30b: Zweites dentales Aufbauteil
- 32b: Trägerbereich
- 34b: Verbindungsbereich
- 36b: Zylindrischer Wandabschnitt
- 38b: Rotationssicherung
- 40b: Vorsprung
- 44: Kegelstumpfförmiger Wandabschnitt

## Patentansprüche

1. Dentales Implantatsystem, umfassend ein Dentalimplantat (10) mit einer Aufnahme (16) für dentale Aufbauteile, wobei sich die Aufnahme (16) entlang einer Längsachse des Dentalimplantats (10) erstreckt, und mindestens ein erstes und mindestens ein zweites dentales Aufbauteil (30a, 30b), die wahlweise mit dem Dentalimplantat (10) verbindbar sind, wobei die Aufbauteile (30a, 30b) jeweils einen Verbindungsbereich (34a, 34b) umfassen, der axial in die Aufnahme (16) einführbar ist, wobei
- die Aufnahme (16) mindestens einen zylindrischen Wandabschnitt (20) umfasst und das Dentalimplantat (10) eine zur Längsachse senkrechte Auflagefläche (26) aufweist, so dass sich ein korrespondierendes erstes Aufbauteil (30a) radial an dem mindestens einen zylindrischen Wandabschnitt (20) und axial an der Auflagefläche (26) abstützen kann;
- die Aufnahme (16) ferner mindestens einen kegelstumpfförmigen Wandabschnitt (18) umfasst, an dem sich ein korrespondierendes zweites Aufbauteil (30b) radial und axial abstützen kann;
- der Verbindungsbereich (34a) des mindestens einen ersten Aufbauteils (30a) mindestens einen zylindrischen Wandabschnitt (36a) und eine zur Längsachse senkrechte Auflagefläche (42) umfasst, so dass die korrespondierenden zylindrischen Wandabschnitte (20, 36a) der Aufnahme (16) und des Verbindungsbereichs (34a) formschlüssig ineinander greifen und die korrespondierenden Auflageflächen (26, 42) aneinander anliegen, wenn das erste Aufbauteil (30a) mit dem Dentalimplantat (10) verbunden wird;
- der Verbindungsbereich (34b) des mindestens einen zweiten Aufbauteils (30b) mindestens einen kegelstumpfförmigen Wandabschnitt (44) umfasst, so dass die korrespondierenden kegelstumpfförmigen Wandabschnitte (18, 44) der Aufnahme (16) und des Verbindungsbereichs (34b) formschlüssig ineinander greifen, wenn das zweite Aufbauteil (30b) mit dem Dentalimplantat (10) verbunden wird; und
- die Aufnahme (16) einen als Rotationssicherung dienenden Abschnitt (22) umfasst, in den ein korrespondierender Abschnitt (38a, 38b) des ersten und/oder zweiten dentalen Aufbauteils (30a, 30b) eingreifen kann,
**dadurch gekennzeichnet, dass** der als Rotationssicherung dienende Abschnitt (22) zwischen einem zylindrischen und einem kegelstumpfförmigen Wandabschnitt (20, 18) angeordnet ist.

2. Dentales Implantatsystem nach Anspruch 1, wobei die zylindrischen und kegelstumpfförmigen Wandabschnitte (20, 18) jeweils rotationssymmetrisch in Bezug auf die Längsachse ausgebildet und in Axialrichtung aufeinander folgend angeordnet sind.

3. Dentales Implantatsystem nach Anspruch 1 oder 2, wobei die Aufnahme (16) einen zylindrischen Wandabschnitt (20) im Bereich ihres apikalen Endes und einen kegelstumpfförmigen Wandabschnitt (18) im Bereich ihres koronalen Endes umfasst.

4. Dentales Implantatsystem nach einem der vorangehenden Ansprüche, wobei sich die senkrechte Auflagefläche (26) vom koronalen Ende der Aufnahme nach außen (16) erstreckt.

5. Dentales Implantatsystem nach einem der Ansprüche 1 bis 3, wobei die senkrechte Auflagefläche (26) durch eine apikale Bodenfläche der Aufnahme (16) gebildet wird.

6. Dentales Implantatsystem nach einem der vorangehenden Ansprüche, wobei die Aufnahme zwei zylindrische Wandabschnitte umfasst, zwischen denen ein kegelstumpfförmiger Wandabschnitt angeordnet ist.

7. Dentales Implantatsystem nach einem der vorangehenden Ansprüche, wobei die Aufnahme zwei kegelstumpfförmige Wandabschnitte umfasst, zwischen denen ein zylindrischer Wandabschnitt angeordnet ist.

8. Dentales Implantatsystem nach Anspruch 7, wobei die zwei kegelstumpfförmigen Wandabschnitte auf derselben gedachten Kegelfläche liegen.

9. Dentales Implantatsystem nach einem der vorangehenden Ansprüche, wobei das Dentalimplantat eine sich apikal an die Aufnahme (16) anschließende Gewindebohrung umfasst, in die eine Schraube zur Fixierung eines Aufbauteils (30a, 30b) eingeschraubt werden kann.

10. Dentales Implantatsystem nach einem der vorangehenden Ansprüche , wobei der Verbindungsabschnitt (34b) des mindestens einen zweiten Aufbauteils (30b) zusätzlich einen zylindrischen Wandabschnitt (36b) umfasst, der mit dem korrespondierenden zylindrischen Wandabschnitte (20) der Aufnahme formschlüssig ineinander greift, wenn das Aufbauteil (30b) mit dem Dentalimplantat (10) verbunden wird.

11. Dentales Implantatsystem nach einem der vorangehenden Ansprüche, wobei der Verbindungsbereich des mindestens einen ersten Aufbauteils zwei zylindrische Wandabschnitte umfasst und/oder wobei der Verbindungsbereich des mindestens einen zweiten Aufbauteils zwei kegelstumpfförmige Wandabschnitte umfasst.

12. Dentales Implantatsystem nach einem der vorangehenden Ansprüche, wobei der Verbindungsbereich (34a, 34b) des mindestens einen ersten Aufbauteils (30a) und/oder des mindestens einen zweiten Aufbauteils (30b) jeweils einen als Rotationssicherung dienenden Abschnitt (38a, 38b) umfasst, der mit einem korrespondierenden Abschnitt (22) der Aufnahme (16) des Dentalimplantats (10) formschlüssig ineinander greift, wenn das erste oder das zweite Aufbauteil (30a, 30b) mit dem Dentalimplantat (10) verbunden wird.

## Claims

1. Dental implant system, comprising a dental implant (10) with a receiver (16) for dental mounting parts, wherein the receiver (16) extends along a longitudinal axis of the dental implant (10), and at least one first and at least one second dental mounting part (30a, 30b), which can alternatively be connected with the dental implant (10), wherein the mounting parts (30a, 30b) in each case comprise a connecting region (34a, 34b), which can be axially introduced into the receiver (16), wherein
- the receiver (16) comprises at least one cylindrical wall portion (20) and the dental implant (10) has a support face (26) perpendicular to the longitudinal axis, so that a corresponding first mounting part (30a) can be supported radially on the at least one cylindrical wall portion (20) and axially on the support face (26);
- the receiver (16) furthermore comprises at least one truncated-cone-shaped wall portion (18), on which a corresponding second mounting part (30b) can be supported radially and axially;
- the connecting region (34a) of the at least one first mounting part (30a) comprises at least one cylindrical wall portion (36a) and a support face (42) perpendicular to the longitudinal axis, so that the corresponding cylindrical wall portions (20, 36a) of the receiver (16) and of the connecting region (34a) engage one another in shape-locking manner and the corresponding support faces (26, 42) abut one another when the first mounting part (30a) is connected to the dental implant (10);
- the connecting region (34b) of the at least one second mounting part (30b) comprises at least one truncated-cone-shaped wall portion (44), so that the corresponding truncated-cone-shaped wall portions (18, 44) of the receiver (16) and of the connecting region (34b) engage one another in shape-locking manner when the second mounting part (30b) is connected to the dental implant (10); and
- the receiver (16) comprises a portion (22) serving as an anti-rotation device, with which a corresponding portion (38a, 38b) of the first and/or second dental mounting part (30a, 30b) can engage,
**characterized in that** the portion (22) serving as an anti-rotation device is arranged between a cylindrical and a truncated-cone-shaped wall portion (20, 18).

2. Dental implant system according to claim 1, wherein the cylindrical and truncated-cone-shaped wall portions (20, 18) are in each case configured rotationally symmetrically in relation to the longitudinal axis and are arranged consecutively in the axial direction.

3. Dental implant system according to claim 1 or 2, wherein the receiver (16) comprises a cylindrical wall portion (20) in the region of its apical end and a truncated-cone-shaped wall portion (18) in the region of its coronal end.

4. Dental implant system according to any one of the preceding claims, wherein the perpendicular support face (26) extends outwardly from the coronal end of the receiver (16).

5. Dental implant system according to any one of claims 1 to 3, wherein the perpendicular support face (26) is formed by an apical base face of the receiver (16).

6. Dental implant system according to any one of the preceding claims, wherein the receiver comprises two cylindrical wall portions, between which a truncated-cone-shaped wall portion is arranged.

7. Dental implant system according to any one of the preceding claims, wherein the receiver comprises two truncated-cone-shaped wall portions, between which a cylindrical wall portion is arranged.

8. Dental implant according to claim 7, wherein the two truncated-cone-shaped wall portions lie on the same imaginary conical face.

9. Dental implant system according to any one of the preceding claims, wherein the dental implant comprises a threaded bore, which apically adjoins the receiver (16) and into which a screw can be screwed to fix a mounting part (30a, 30b).

10. Dental implant system according to any one of the preceding claims, wherein the connecting portion (34b) of the at least one second mounting part (30b) additionally comprises a cylindrical wall portion (36b) mutually engaging with the corresponding cylindrical wall portion (20) of the receiver in shape-locking manner when the mounting part (30b) is connected to the dental implant (10).

11. Dental implant system according to any one of the preceding claims, wherein the connecting region of the at least one first mounting part comprises two cylindrical wall portions and/or wherein the connecting region of the at least one second mounting part comprises two truncated-cone-shaped wall portions.

12. Dental implant system according to any one of the preceding claims, wherein the connecting region (34a, 34b) of the at least one first mounting part (30a) and/or of the at least one second mounting part (30b) in each case comprises a portion (38a, 38b) serving as an anti-rotation device and mutually engaging with a corresponding portion (22) of the receiver (16) of the dental implant (10) in shape-locking manner when the first or the second mounting part (30a, 30b) is connected to the dental implant (10).

## Revendications

1. Système d'implant dentaire, comprenant un implant dentaire (10) comportant un logement (16) pour des éléments structurels dentaires, le logement (16) s'étendant le long d'un axe longitudinal de l'implant dentaire (10), et au moins un premier et au moins un deuxième élément structurel dentaire (30a, 30b) pouvant être reliés, au choix, à l'implant dentaire (10), les éléments structurels (30a, 30b) comprenant chacun une zone de liaison (34a, 34b) pouvant être introduite axialement dans le logement (16), dans lequel
- le logement (16) comprend au moins un tronçon de paroi cylindrique (20) et l'implant dentaire (10) présente une face d'appui (26) perpendiculaire à l'axe longitudinal, de sorte qu'un premier élément structurel (30a) correspondant peut s'appuyer radialement contre l'au moins un tronçon de paroi cylindrique (20) et axialement contre la face d'appui (26) ;
- de plus, le logement (16) présente au moins un tronçon de paroi (18) de forme tronconique contre lequel un deuxième élément structurel (30b) correspondant peut s'appuyer radialement et axialement ;
- la zone de liaison (34a) de l'au moins un premier élément structurel (30a) comprend au moins un tronçon de paroi cylindrique (36a) et une face d'appui (42) perpendiculaire à l'axe longitudinal, de sorte que les tronçons de paroi cylindriques (20, 36a) correspondants du logement (16) et de la zone de liaison (34a) s'interpénètrent par complémentarité de forme et les faces d'appui (26, 42) correspondantes sont côte à côte lorsque le premier élément structurel (30a) est relié à l'implant dentaire (10) ;
- la zone de liaison (34b) de l'au moins un deuxième élément structurel (30b) comprend au moins un tronçon de paroi tronconique (44), de sorte que les tronçons de paroi tronconiques (18, 44) correspondants du logement (16) et de la zone de liaison (34b) s'interpénètrent par complémentarité de forme lorsque le deuxième élément structurel (30b) est relié à l'implant dentaire (10) ; et
- le logement (16) comprend un tronçon (22) servant à la protection en rotation, dans lequel un tronçon (38a, 38b) correspondant du premier et/ou du deuxième élément structurel dentaire (30a, 30b) peut pénétrer,
**caractérisé en ce que** le tronçon (22), servant de protection en rotation, est disposé entre un tronçon de paroi cylindrique (20) et un tronçon de paroi tronconique (18).

2. Système d'implant dentaire selon la revendication 1, dans lequel les tronçons de paroi cylindriques et tronconiques (20, 18) sont chacun symétriques en rotation par rapport à l'axe longitudinal et sont disposés de façon consécutive dans le sens axial.

3. Système d'implant dentaire selon la revendication 1 ou la revendication 2, dans lequel le logement (16) comprend un tronçon de paroi cylindrique (20) au niveau de son extrémité apicale et un tronçon de paroi tronconique (18) au niveau de son extrémité coronale.

4. Système d'implant dentaire selon l'une quelconque des revendications précédentes, dans lequel la face d'appui perpendiculaire (26) s'étend de l'extrémité coronale du logement vers l'extérieur (16).

5. Système d'implant dentaire selon l'une quelconque des revendications 1 à 3, dans lequel la face d'appui perpendiculaire (26) est formée par un fond apical du logement (16).

6. Système d'implant dentaire selon l'une quelconque des revendications précédentes, dans lequel le logement comprend deux tronçons de paroi cylindriques entre lesquels est disposé un tronçon de paroi tronconique.

7. Système d'implant dentaire selon l'une quelconque des revendications précédentes, dans lequel le logement comprend deux tronçons de paroi tronconiques entre lesquels est disposé un tronçon de paroi cylindrique.

8. Système d'implant dentaire selon la revendication 7, dans lequel les deux tronçons de paroi tronconiques se trouvent sur la même face conique virtuelle.

9. Système d'implant dentaire selon l'une quelconque des revendications précédentes, dans lequel l'implant dentaire comprend un trou fileté adjacent, dans la zone apicale, au logement (16), trou dans lequel une vis peut être vissée pour immobiliser un élément structurel (30a, 30b).

10. Système d'implant dentaire selon l'une quelconque des revendications précédentes, dans lequel le tronçon de liaison (34b) de l'au moins un deuxième élément structurel (30b) comprend en plus un tronçon de paroi cylindrique (36b) qui s'interpénètre par complémentarité de forme avec les tronçons de paroi cylindriques (20) correspondants lorsque l'élément structurel (30b) est relié à l'implant dentaire (10).

11. Système d'implant dentaire selon l'une quelconque des revendications précédentes, dans lequel la zone de liaison de l'au moins un premier élément structurel comprend deux tronçons de paroi cylindriques et/ou dans lequel la zone de liaison de l'au moins un deuxième élément structurel comprend deux tronçons de paroi tronconiques.

12. Système d'implant dentaire selon l'une quelconque des revendications précédentes, dans lequel la zone de liaison (34a, 34b) de l'au moins un premier élément structurel (30a) et/ou de l'au moins un deuxième élément structurel (30b) comprend un tronçon (38a, 38b) servant de protection en rotation qui s'interpénètre par complémentarité de forme avec un tronçon correspondant (22) du logement (16) de l'implant dentaire (10) lorsque le premier ou le deuxième élément structurel (30a, 30b) est relié à un implant dentaire (10).
